# FASCICULE DE BREVET EUROPEEN

(11) **EP 0 894 484 B1**
(45) Date de publication et mention de la délivrance du brevet: **17.10.2007**
(21) Numéro de dépôt: 98440163.8
(22) Date de dépôt: 29.07.1998
(51) Int. Cl.: A61F 11/08

(54) **Valve acoustique apte à opérer un filtrage sélectif et non linéaire du son**
Lärmventil zum selectiven und non-linearen einstellen der Geräuschdurchlässigkeit
Acoustic valve able to effect a selective and non-linear filtering of sounds

(30) Priorité: 30.07.1997 FR 9709925
(43) Date de publication de la demande: 03.02.1999
(73) Titulaire: I.S.L. Institut Franco-Allemand de Recherches de Saint-Louis, 68301 Saint-Louis Cedex (FR)
(72) Inventeur: Hamery, Pascal, 68400 Riediesheim (FR); Dancer, Armand, 39100 Authume (FR); Evrard, Georges, 68480 Durmenach (FR)
(74) Mandataire: Somnier, Jean-Louis

(56) Documents cités:
- EP-A- 0 112 594
- EP-A- 0 440 572
- FR-A- 2 676 642
- GB-A- 643 927
- US-A- 3 730 181
- US-A- 4 540 063
- US-A- 5 467 784

## Description

La présente invention a pour objet un dispositif acoustique apte à opérer un filtrage sélectif et non linéaire du son, et à être mis en place dans le conduit auditif externe d'un utilisateur par l'intermédiaire d'un bouchon d'oreille, en vue d'une utilisation notamment dans un environnement militaire ou industriel.

Un des problèmes à résoudre pour les militaires est de communiquer entre eux, de détecter, localiser et identifier les sources de bruits extérieurs, tout en protégeant leur ouïe contre les bruits impulsionnels, notamment les bruits d'armes, de forts niveaux (jusqu'à 190dB SPL).

Il existe déjà des bouchons que l'on introduit dans le canal auditif et qui atténuent fortement et de la même manière tous les bruits, qu'il s'agisse de bruits impulsionnels de forts niveaux, de bruits d'environnement ou de sons de parole de faibles niveaux.

Le document FR-A-2050740 décrit un filtre contre le bruit, sensible à un degré élevé à l'énergie du langage et à un degré moindre au bruit de fond, comprenant essentiellement une capsule adaptée à être insérée dans le conduit auditif d'un utilisateur, et un élément vibrant apte à discriminer les fréquences, positionné à l'intérieur de ladite capsule, le filtre comportant en outre un diaphragme couplé de façon opérante à l'élément vibrant.

Cependant ce dispositif est de conception complexe et d'un prix de revient élevé. En outre les courbes d'atténuation obtenues à partir de ce dispositif ne sont pas idéales dans le contexte environnemental militaire, car il atténue de la même manière les bruits de faibles et de forts niveaux.

Le document EP-A-0440572 a pour objet un dispositif de transmission du son à filtrage sélectif, destiné à être mis en place dans le conduit auditif externe et comprenant d'une part un embout, réalisé par moulage à partir d'une empreinte prise sur le porteur et obturant complètement le conduit auditif externe, et d'autre part une valve acoustique insérée dans cet embout. Le dispositif comporte au moins une valve acoustique placée à l'extrémité d'un tube, et d'une section sensiblement supérieure à celle de ce tube. Cette valve joue le rôle de cavité de résonance, selon le principe du résonateur de HELMOLTZ, le filtre ainsi constitué étant un filtre de quatrième ordre dont la pente d'atténuation est de 30 décibels par octave.

Toutefois ce dispositif est relativement complexe au niveau de sa conception et présente un problème de résistance capillaire du fait de la propagation des ondes acoustiques lors de leur passage de la valve dans le tube, de plus petit diamètre, qui se prolonge dans la cavité résiduelle. Il en résulte que ce dispositif de filtre acoustique atténue de manière sensiblement identique l'intensité des bruits de faibles niveaux (voix humaine, bruits d'environnement) et de forts niveaux (bruits impulsionnels du type bruits d'armes).

La présente invention a pour but de remédier à ces inconvénients en proposant une valve acoustique d'une plus grande simplicité de conception, permettant d'améliorer les performances de filtrage obtenues par les dispositifs connus, notamment au niveau du comportement acoustique non linéaire du filtre. De plus elle est particulièrement adaptée à protéger le tympan de l'utilisateur contre des impulsions sonores d'intensité élevée, tout en laissant passer les ondes sonores intelligibles représentatives de la parole humaine et les bruits d'environnement de faibles niveaux.

La valve acoustique selon l'invention a été mise au point après de nombreuses études et expérimentations visant à obtenir un comportement non linéaire efficace, et elle se caractérise en ce qu'elle est apte à opérer un filtrage non linéaire du son et qu'elle consiste en un tube et deux disques rigides perforés espacés axialement l'un de l'autre d'une distance comprise entre 2 et 7mm; la surface totale perforée de chaque disque étant comprise entre 0.03 et 0.5mm2.

Selon une caractéristique additionnelle de l'invention l'épaisseur des disques dans la zone de perforation est avantageusement comprise entre 0.05 et 0.5mm afin d'éviter l'apparition d'un phénomène de résistance capillaire.

Dans un mode de réalisation préférentiel de l'invention la valve acoustique est constituée de deux parties cylindriques creuses fermées chacune à l'une de leurs extrémités par un disque comportant un orifice central et ouverte à leur autre extrémité dont le bord périphérique comporte un épaulement permettant d'assembler les deux parties par lesdites extrémités ouvertes complémentaires, solidarisées par un moyen de fixation.

Dans d'autres modes de réalisation de l'invention l'un des disques, ou les deux, peut être positionné à distance de l'extrémité du tube.

La valve acoustique selon l'invention est préférentiellement réalisée par moulage d'une matière plastique rigide telle que l'"ALTUGLAS"^{®} (marque déposée) ou une résine époxy.

Les avantages et les caractéristiques de la présente invention ressortiront plus clairement de la description qui suit et qui se rapporte au dessin annexé, fourni à titre de simple illustration de l'invention, vis-à-vis de laquelle il ne présente aucun caractère limitatif.

Dans le dessin annexé:
- la figure 1 représente une vue en perspective d'un premier mode de réalisation de la valve acoustique selon l'invention.
- la figure 2 représente une vue en coupe longitudinale de la même valve acoustique.
- la figure 3a représente une vue schématique en coupe longitudinale d'un deuxième mode de réalisation de la valve selon l'invention.
- la figure 3b représente une vue schématique en coupe longitudinale d'un troisième mode de réalisation de la valve selon l'invention.

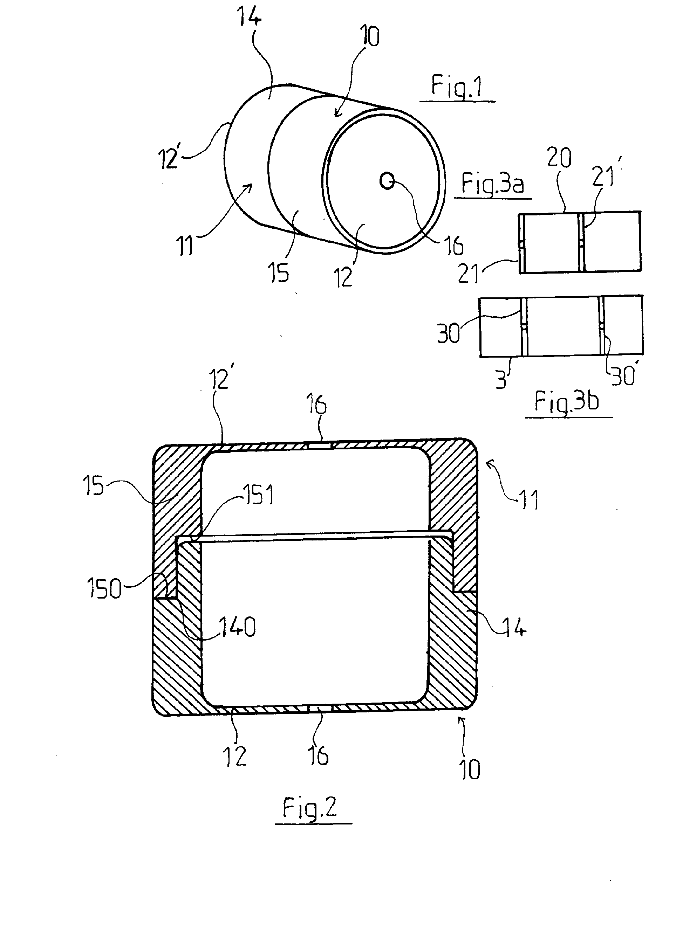

Si on se réfère aux figures 1 et 2 on peut voir que dans un premier mode de réalisation la valve acoustique selon l'invention présente une forme cylindrique et comprend deux pièces tubulaires 10 et 11 sensiblement de mêmes dimensions.

Chaque pièce 10,11 est réalisée par moulage en une matière plastique moulée du type altuglass^{®} ou résine époxy et comporte un disque 12,12' formant l'extrémité d'un cylindre creux 14,15 ouvert à son autre extrémité et dont le bord périphérique 140,150 comporte un épaulement 141,151, les deux épaulements 141 et 151 étant complémentaires l'un de l'autre afin de permettre l'assemblage des deux pièces 10,11 par emboîtement et par collage.

Chaque disque 12,12' présente un diamètre compris entre 2 et 4mm, et comporte centralement un orifice circulaire 16 dont le diamètre est compris entre 0.2 et 0.6mm.

Les dimensions des pièces 10 et 11 sont déterminées de manière que la distance séparant les deux disques 12 et 12' après assemblage des pièces 10,11, soit comprise entre 2 et 7mm.

Les disques 12 et 12' pourraient également comporter une pluralité de perçages, dont le diamètre serait déterminé de manière que la surface totale des perçages soit sensiblement égale à celle du perçage central 16.

Si on se réfère maintenant à la figure 3a on peut voir que dans un deuxième mode de réalisation de l'invention la valve est constituée d'un tube 20 renfermant deux disques 21 et 21', percés centralement et sensiblement de même diamètre que le diamètre interne du tube 20, dont l'un 21 est positionné à l'une des extrémités du tube 20 et l'autre 21' en retrait de l'extrémité opposée.

Si on se réfère à la figure 3b on peut voir que dans un troisième mode de réalisation la valve acoustique comprend un tube 3 renfermant deux disques 30 et 30' percés centralement et disposés chacun en retrait de l'une des extrémités du tube 3.

Dans chacun de ces deux derniers modes de réalisation, les dimensions des disques et la distance les séparant axialement se situent dans les mêmes limites que celles du premier mode de réalisation.

La valve acoustique selon l'invention est destinée à être insérée dans un bouchon d'oreille perforé, réalisé de préférence en matériau élastique, en vue de sa mise en place dans le conduit auditif externe d'un utilisateur.

Les dimensions, ci-dessus définies,de la valve acoustique selon l'invention permettent d'obtenir une atténuation non linéaire efficace, l'atténuation du son augmentant avec le niveau de bruit extérieur de manière instantanée, notamment au dessus d'un niveau de bruit de 110 dB SPL.

La valve acoustique selon l'invention permet aux utilisateurs de communiquer entre eux en localisant et en identifiant les sources sonores représentatives de la parole humaine et les bruits de faibles niveaux dans l'environnement, en atténuant les bruits impulsionnels, par exemple les bruits d'armes à feu de tous calibres ou de machines industrielles telles que les presses métallurgiques.

## Revendications

1. Valve acoustique apte à être mise en place dans un bouchon d'oreille perforé et apte à opérer un filtrage sélectif du son, **caractérisée en ce qu'**elle est apte à opérer un filtrage non linéaire du son et consiste en un tube (10,20,3) et deux disques rigides perforés (12,12';21,21';30,30') espacés axialement l'un de l'autre d'une distance comprise entre 2 et 7 mm; la surface totale perforée de chaque disque (12,12';21,21';30,30') étant comprise entre 0,03 et 0,5 mm².

2. Valve acoustique selon la revendication 1, **caractérisée en ce que** l'un (21) des deux disques rigides (21,21') est positionné à l'une des extrémités du tube (20), l'autre (21') l'étant en retrait de l'extrémité opposée.

3. Valve acoustique selon la revendication 1, **caractérisée en ce que** chaque disque rigide (30,30') est disposé en retrait de l'une des extrémités du tube (3).

4. Valve acoustique selon la revendication 1, **caractérisée en ce qu'**elle est constituée de deux parties cylindriques creuses (14,15) fermées chacune à l'une de leurs extrémités par le disque (12,12') correspondant et ouverte à leur autre extrémité dont le bord périphérique (140,150) comporte un épaulement (141,151) permettant d'assembler les deux parties (14,15) par lesdites extrémités ouvertes complémentaires, solidarisées par un moyen de fixation.

5. Valve acoustique selon l'une des revendications 1 à 4, **caractérisée en ce qu'**elle est réalisée par moulage d'une matière plastique rigide.

6. Valve acoustique selon l'une des revendications 1 à 5, **caractérisée en ce que** l'épaisseur des disques (12,12') dans les zones de perforation est comprise entre 0,05 et 0,5 mm.

## Claims

1. An acoustic valve suitable for insertion into a perforated ear plug and capable of performing a selective filtering of sound, **characterised in that** it is capable of performing a non-linear filtering of sound and consists of a tube (10, 20, 3) and two perforated rigid discs (12, 12'; 21, 21'; 30, 30') that are spaced apart axially from one each other at a distance of between 2 and 7 mm, the total perforated surface of each disc (12, 12'; 21, 21'; 30, 30') being between 0.03 and 0.5 mm².

2. Acoustic valve according to claim 1, **characterised in that** one (21) of the two rigid discs (21, 21') is positioned at one of the ends of the tube (20), the other (21') is set back from the opposite end.

3. Acoustic valve according to claim 1, **characterised in that** each rigid disc (30, 30') is arranged set back from one of the ends of the tube (3).

4. Acoustic valve according to claim 1, **characterised in that** it consists of two hollow cylindrical parts (14, 15) each closed at one of their ends by the corresponding disc (12, 12') and open at their other end, the peripheral edge (140, 150) of which comprises a shoulder (141, 151) making it possible to assemble the two parts (14, 15) via the said open complementary ends, joined together by a securing means.

5. Acoustic valve according to one of claims 1 to 4, **characterised in that** it is produced by moulding from a rigid plastic material.

6. Acoustic valve according to one of claims 1 to 5, **characterised in that** the thickness of the discs (12, 12') in the perforation zones is between 0.05 and 0.5 mm.

## Patentansprüche

1. Akustisches Ventil, das in einem perforierten Ohrstöpsel angebracht werden kann und dazu geeignet ist, eine selektive Filterung des Schalls vorzunehmen, **dadurch gekennzeichnet, dass** es dazu geeignet ist, eine nicht lineare Filterung des Schalls vorzunehmen und **dadurch**, dass es ein Rohr (10, 20, 3) und zwei starre perforierte Scheiben (12, 12'; 21, 21'; 30, 30') umfasst, die axial einen Abstand zwischen 2 und 7mm voneinander entfernt sind; wobei die gesamte perforierte Fläche jeder Scheibe (12, 12'; 21, 21'; 30, 30') zwischen 0,03 und 0,5 mm² beträgt.

2. Akustisches Ventil nach Anspruch 1, **dadurch gekennzeichnet, dass** eine (21) der zwei starren Scheiben (21, 21') an einem der Enden des Rohrs (20) und die andere (21') vom gegenüberliegenden Ende zurückversetzt positioniert ist.

3. Akustisches Ventil nach Anspruch 1, **dadurch gekennzeichnet, dass** jede starre Scheibe (30, 30') von einem der Enden des Rohrs (3) zurückversetzt angebracht ist.

4. Akustisches Ventil nach Anspruch 1, **dadurch gekennzeichnet, dass** es zwei hohle zylindrische Teile (14, 15) umfasst, die jeweils an einem ihrer Enden durch die entsprechende Scheibe (12, 12') geschlossen sind und an ihrem anderen Ende geöffnet sind, dessen Umfangskante (140, 150) einen Absatz (141, 151) aufweist, der es ermöglicht, die zwei Teile (14, 15) durch die komplementären offenen Enden, durch ein Befestigungsmittel miteinander verbunden, zusammenzufügen.

5. Akustisches Ventil nach einem der Ansprüche 1 bis 4, **dadurch gekennzeichnet, dass** es durch Giessen eines starren Kunststoffmaterials hergestellt ist.

6. Akustisches Ventil nach einem der Ansprüche 1 bis 5, **dadurch gekennzeichnet, dass** die Dicke der Scheiben (12, 12') in den Bereichen der Perforation zwischen 0,05 und 0,5mm liegt.
